# FASCICULE DE BREVET EUROPEEN

(11) **EP 1 274 989 B1**
(45) Date de publication et mention de la délivrance du brevet: **01.09.2010**
(21) Numéro de dépôt: 00985379.7
(22) Date de dépôt: 01.12.2000
(51) Int. Cl.: G01N 23/04, A61B 6/00

(54) **PROCEDE D'AMELIORATION D'UN EXAMEN RADIOLOGIQUE ET DISPOSITIF POUR LA MISE EN OEUVRE DE CE PROCEDE**
VERFAHREN ZUR VERBESSERUNG EINER RÖNTGENSTRAHLENUNTERSUCHUNG UND ZUGEHÖRIGE VORRICHTUNG
METHOD FOR IMPROVING A RADIOLOGICAL EXAMINATION AND DEVICE THEREFOR

(30) Priorité: 03.12.1999 FR 9915273
(43) Date de publication de la demande: 15.01.2003
(73) Titulaire: Commissariat à l'Énergie Atomique et aux Énergies Alternatives, 75015 Paris (FR)
(72) Inventeur: DINTEN, Jean-Marc, F-69008 Lyon (FR); ROBERT-COUTANT, Christine, F-38410 Saint-Martin D'Uriage (FR)
(74) Mandataire: Poulin, Gérard
(86) Numéro de dépôt international: PCT/FR2000/003357
(87) Numéro de publication internationale: WO 2001/040754

(56) Documents cités:
- WO-A-96/35372
- US-A- 4 773 087
- US-A- 5 150 394
- US-A- 5 457 724

## Description

### Domaine technique

La présente invention concerne un procédé d'amélioration d'un examen radiologique d'une zone d'un corps ainsi qu'un dispositif pour la mise en oeuvre de ce procédé.

Par « corps » on entend aussi bien un objet (par exemple un tableau ou une momie) qu'une personne ou même un animal.

L'invention s'applique à tout examen radiologique utilisant un détecteur bidimensionnel de rayons X et, en particulier, à l'ostéodensitométrie par rayonnement X bi-énergie à faisceau conique.

L'invention concerne plus particulièrement le positionnement d'un patient préalablement à un tel examen radiologique ainsi que le réglage de la dose de rayons X à transmettre à ce patient lors de cet examen.

### État de la technique antérieure

On rappelle que l'ostéodensitométrie par rayons X est une technique de mesure de masses et de densités osseuses à partir d'acquisitions radiographiques effectuées à une pluralité d'énergies.

On utilise en général deux énergies que l'on appelle respectivement "haute énergie" et "basse énergie".

On distingue trois familles de systèmes d'ostéodensitométrie :
- les systèmes à pinceau dé rayonnement ("pencil beam systems") qui utilisent une source de rayons X collimatée par un trou et un monodétecteur de rayons X qui est également collimaté,
- les systèmes à faisceau en éventail ("fan beam systems") qui utilisent une source de rayons X collimatée par une fente et un détecteur linéaire de rayons X, et
- les systèmes à faisceau conique ("cone beam systems") qui utilisent une source de rayons X non collimatée et un détecteur bidimensionnel de rayons X.

Les systèmes des deux premières familles nécessitent un balayage mécanique pour obtenir une image globale d'une zone anatomique alors que les systèmes de la troisième famille permettent d'établir directement une image complète.

C'est pourquoi l'invention concerne plus particulièrement les systèmes d'ostéodensitométrie par rayonnement X bi-énergie à faisceau conique.

Les principes méthodologiques de l'ostéodensitométrie par rayonnement X bi-énergie et les principales solutions techniques actuellement utilisées sont connus par les deux documents suivants auxquels on se reportera :
[1] "Technical Principles of Dual Energy X-Ray Absorptiometry", G.M. Blake et I. Fogelman, Seminars in Nuclear Medicine, Vol XXVII, n°3, juillet 1997, pages 210 à 228 et
[2] "The Evaluation of Osteoporosis : Dual Energy X-Ray Absorptiometry and Ultrasound in Clinical Practice", Second Edition, G.M. Blake, H.W. Wahner et I. Fogelman, Martin Dunitz Editor, 1999, ISBN 1-85317-472-6.

On se reportera plus particulièrement aux chapitres 3, 4 et 5 du document [2] où sont décrits les principes de mesure de densités osseuses à deux énergies et les systèmes connus pour faire de telles mesures.

On connaît aussi des systèmes d'ostéodensitométrie de zones bidimensionnelles par les documents suivants auxquels on se reportera :
[3] brevet US-5,150,394 du 22 septembre 1992, "Dual-Energy System for Quantitative Radiographic Imaging", (Andrew Karellas), et
[4] Demande internationale publiée le 14 novembre 1996, n° de publication WO 96/35372, "A System for Quantitative Radiographic Imaging", (Andrew Karellas).

On donne en outre les précisions suivantes :

En ce qui concerne le positionnement du patient, sur les systèmes de type "pencil beam" ou "fan beam", un premier positionnement du patient s'effectue à l'aide d'un pointeur laser qui repère la zone d'examen à partir d'observations morphologiques externes. Ensuite, le balayage avec rayonnement X débute. Si le patient est bien positionné l'examen se poursuit mais si à l'observation sur l'écran des premières lignes acquises le positionnement n'est pas bon, l'opérateur arrête tout et effectue un nouveau positionnement puis relance l'examen.

A ce sujet, on se reportera au document [2] pages 198 à 200 pour ce qui concerne la colonne vertébrale et aux pages 265 à 267 pour ce qui concerne la hanche.

Une étude récente a montré que, pour des systèmes du genre "pencil beam", le repositionnement avait lieu dans 50% des cas et que, pour environ 10% des examens, il fallait repositionner jusqu'à trois fois le patient. A ce sujet, on se reportera au document suivant :
[5] Insights, vol. 10, n°1, mars 1999, pages 10 et 11 (revue éditée par la Société Hologic), "Independent survey reveals surprising, disappointing results for Lunar users".

Sur les systèmes du genre "cone beam" connus, utilisés pour des examens de zones périphériques, le positionnement du patient est assuré par un système mécanique d'aide au positionnement, par exemple une poignée pour l'avant-bras et une cuvette formée pour le talon.

En outre, on connaît déjà la possibilité d'utiliser un cliché préalable à l'examen afin d'obtenir des données de reconnaissance avant balayage (« prescan scout data ») pour « centrer » le patient, dans le domaine de la tomographie, par le brevet suivant :
[6] US 5457724 « Automatic field of view and patient centering determination from prescan scout data » du 10 octobre 1995.

Dans ce brevet US 5457724, avant de reconstruire une coupe tomographique d'un patient, on acquiert deux projections monodimensionnelles (à l'aide d'un faisceau en éventail) à 0° et 90° de cette coupe. On détecte les points correspondant aux bords du patient dans les deux projections, et on en déduit la position du centre de la zone et la taille du champ d'acquisition tomographique. Ces paramètres sont donnés à l'opérateur et peuvent être utilisés pour déplacer le patient afin de mieux le centrer pour l'acquisition tomographique.

Le but est d'obtenir la meilleure qualité d'image reconstruite possible. En effet, comme les systèmes tomographiques sont étudiés pour que l'atténuation maximale se trouve au centre de la zone d'acquisition et comme les corrections de durcissement de spectre dépendent de la taille du champ d'acquisition, la qualité de l'image reconstruite dépend du bon centrage et de la taille du champ d'acquisition.

### Exposé de l'invention

D'une manière générale, la présente invention a pour but d'améliorer la reproductibilité des mesures faites au cours de tout examen radiologique qui utilise un détecteur bidimensionnel.

L'invention a également pour but d'optimiser la dose de rayons X transmise au patient au cours d'un tel examen.

En particulier, la présente invention a pour but d'améliorer un examen d'ostéodensitométrie par rayonnement X bi-énergie à faisceau conique et, plus particulièrement, d'augmenter la reproductibilité des mesures de densité osseuse dans des zones anatomiques d'un patient soumis à un tel examen.

De façon précise, la présente invention a pour objet un procédé d'amélioration d'un examen radiologique d'une zone d'un corps, cet examen radiologique étant effectué au moyen d'un dispositif radiologique comprenant un détecteur bidimensionnel de rayons x, ce procédé étant caractérisé en ce que, avant l'examen radiologique, on fait un cliché bidimensionnel de radioscopie (donc avec une faible dose de rayons X), avec une seule énergie pour ces rayons X, de la zone du corps à examiner et l'on utilise ce cliché pour déterminer des premiers points caractéristiques qui définissent un référentiel de mesure, ainsi que des paramètres géométriques d'un déplacement apte à faire correspondre sensiblement ce référentiel de mesure avec un référentiel préétabli à partir de points caractéristiques équivalents correspondant aux premiers points caractéristiques .

Cet examen radiologique peut être un examen d'ostéodensitométrie.

On précise que les premiers points caractéristiques sont en général des points précisément repérables de l'image, par exemple des points de contour, des points d'inflexion ou des points de densité extrême.

Selon un mode de mise en oeuvre particulier du procédé objet de l'invention, lorsque l'examen radiologique est un premier examen, le référentiel préétabli est un référentiel théorique, adapté au corps examiné.

Selon un autre mode de mise en oeuvre particulier, lorsque l'examen radiologique suit un examen radiologique précédent, effectué au moyen du même dispositif radiologique, le référentiel préétabli est un référentiel qui est défini sur un cliché précédent à partir des points caractéristiques équivalents.

Conformément à la présente invention, le cliché bidimensionnel est utilisable (uniquement ou en plus) pour régler la dose de rayons X à transmettre au corps lors de l'examen radiologique en adaptant le point de fonctionnement du dispositif radiologique à la morphologie du corps à examiner ou en ajustant, à la morphologie du corps à examiner, la zone à irradier par les rayons X lors de l'examen.

La présente invention concerne en outre un dispositif radiologique pour la mise en oeuvre du procédé objet de l'invention, ce dispositif comprenant :
- une source de rayons X, apte à fournir un faisceau conique de rayons X à au moins une énergie,
- des moyens de variation de la dose de rayons X susceptible d'être reçue par le corps à examiner,
- un détecteur bidimensionnel de rayons X, qui est disposé parallèlement à un plan défini par deux directions orthogonales et qui est perpendiculaire à l'axe du faisceau de rayons X,
- des moyens de support du corps à examiner, ces moyens de support étant transparents aux rayons X émis par la source, disposés entre la source et le détecteur et susceptibles de subir un déplacement relatif par rapport à l'ensemble formé par la source et le détecteur, suivant les deux directions orthogonales,
- des moyens de détermination de premiers points caractéristiques sur un cliché bidimensionnel de radioscopie, ces premiers points définissant un référentiel de mesure, et
- des moyens de calcul, prévus pour déterminer les paramètres géométriques d'un déplacement apte à faire correspondre sensiblement ce référentiel de mesure avec un référentiel préétabli à partir de points caractéristiques équivalents correspondant aux premiers points caractéristiques.

### Brève description des dessins

La présente invention sera mieux comprise à la lecture de la description d'exemples de réalisation donnés ci-après, à titre purement indicatif et nullement limitatif, en faisant référence aux dessins annexés sur lesquels :
- la figure 1 est une vue schématique d'un système d'ostéodensitométrie osseuse par rayonnement X à deux énergies, à faisceau conique, qui est utilisable pour la mise en oeuvre de l'invention, et
- la figure 2 est un organigramme d'une procédure qui est utilisée dans un mode de mise en oeuvre particulier de l'invention.

### Exposé détaillé de modes de réalisation particuliers

On décrit maintenant un exemple de procédé conforme à l'invention, relatif au positionnement d'un patient avant un examen d'une zone anatomique de ce dernier par ostéodensitométrie par rayonnement X bi-énergie à faisceau conique.

Dans cet exemple, avant de faire les acquisitions à haute et basse énergies, on fait un cliché bidimensionnel de radioscopie, à faible dose de rayons X et à une seule énergie, de la zone anatomique du patient.

Ensuite, d'une détection de points caractéristiques définis à partir des contours osseux sur ce cliché, on déduit les paramètres géométriques d'un déplacement permettant de faire correspondre au mieux un référentiel défini par ces points caractéristiques
- soit avec un référentiel « théorique » défini par des points équivalents, choisis à l'avance en fonction de la zone anatomique considérée, dans le cas d'un premier examen,
- soit avec un référentiel défini par des points équivalents, choisis sur un cliché bidimensionnel précédent, dans le cas d'un n^{ième} examen avec n>1.

Dans les deux cas, on effectue un déplacement du patient par rapport au système source-détecteur que comprend le dispositif d'ostéodensitométrie, ou du système source-détecteur par rapport au patient, par commande manuelle ou automatique.

Précisons en outre que dans la présente invention il s'agit de radiologie (avec un détecteur bidimensionnel) et non pas de tomographie (avec un détecteur pour faisceau en éventail, animé d'un mouvement de rotation comme cela est décrit dans le document [6] mentionné plus haut). L'image finale est une projection bidimensionnelle et non pas une coupe reconstruite. De plus, dans l'invention, on utilise un seul cliché préalable et non pas deux clichés à 90° l'un de l'autre.

En outre, un but de l'exemple considéré de l'invention est la reproductibilité de la mesure de masse osseuse calculée à partir de l'image, et pas la qualité de l'image elle-même.

De plus, dans les systèmes tomographiques le recentrage du patient n'est pas automatique.

Plus précisément, ce recentrage est automatique en hauteur, c'est-à-dire perpendiculairement au plan de la table qui supporte le patient ou parallèlement à l'axe du faisceau X, mais il ne l'est pas en largeur, c'est-à-dire suivant la plus petite dimension de cette table, car le déplacement latéral de la table n'est pas prévu ou n'est pas nécessaire.

Rappelons que la reproductibilité est la propriété qu'a le dispositif de mesure de donner la même mesure pour différents examens sur le même patient (supposé de densité osseuse constante) et la même zone anatomique.

Dans le cas d'un patient dont la masse osseuse varie dans le temps, sous l'effet d'une maladie ou d'un traitement par exemple, cette propriété de reproductibilité permet de quantifier ces variations de la masse osseuse.

De plus, dans la présente invention, on peut utiliser le cliché de radioscopie pour
1/ adapter la dose d'irradiation par réglage du flux de rayons X en modifiant le courant appliqué au tube à rayons X utilisé et/ou la tension appliquée à ce tube
2/ positionner automatiquement des caches permettant de limiter la zone d'irradiation, ce qui n'est pas possible en tomographie sous peine de projections tronquées.

De cette façon la dose d'irradiation reçue par le patient est minimisée.

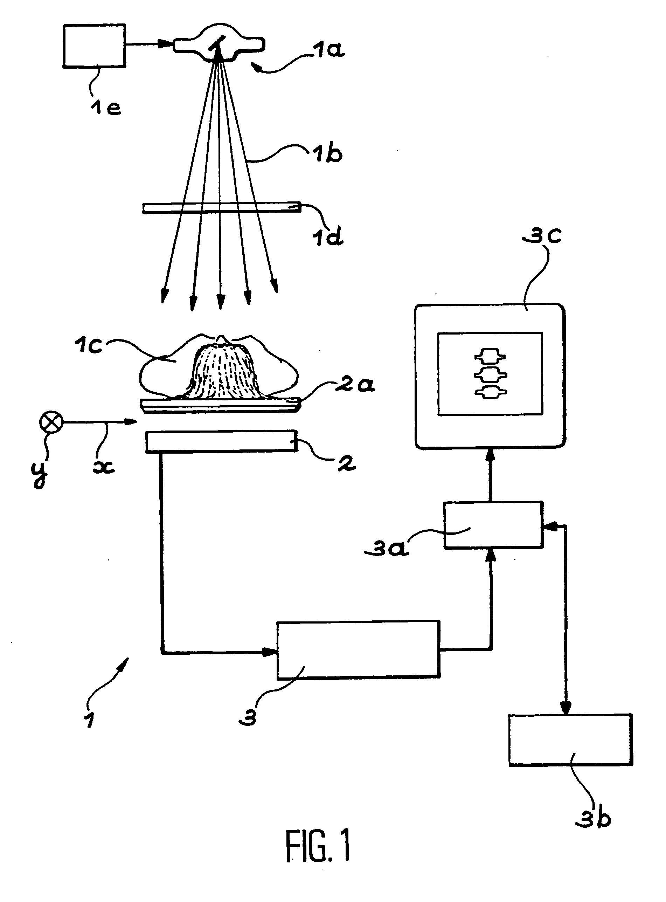

On voit sur la figure 1 un système d'ostéodensitométrie osseuse 1 que l'on utilise aussi pour faire le cliché préalable de radioscopie conformément à l'invention.

Ce système comprend une source 1a de rayons X, apte à envoyer un faisceau conique 1b de rayons X vers le corps d'un patient 1c à examiner. Cette source la est apte à émettre des rayonnements X correspondant respectivement à deux niveaux distincts d'énergie. Ces deux niveaux sont utilisés pour obtenir deux images distinctes du patient.

Un filtre amovible ld est interposable entre la source 1a et le patient 1c et sert à améliorer les qualités spectrales du faisceau.

Le système 1 comprend aussi un détecteur bidimensionnel 2 qui est très schématiquement représenté en coupe transversale sur la figure 1 et destiné à détecter les rayons X émis par la source et ayant traversé le patient 1c.

Ce détecteur 2 est parallèle à un plan défini par deux directions orthogonales x et y et est perpendiculaire à l'axe du faisceau de rayons X.

Le patient est placé sur un support approprié 2a, par exemple un lit, qui est transparent aux rayons X. Dans l'exemple de la figure 1 la source la (munie de l'éventuel filtre 1d) est placée au-dessus du patient reposant sur le support tandis que le détecteur est placé en-dessous de ce support.

Des moyens non représentés sont prévus pour déplacer le support 2a par rapport à la source la et au détecteur 2, qui sont alors fixes, ou sont prévus pour déplacer la source 1a et le détecteur 2 par rapport au support 2a qui est alors fixe, ces déplacements ayant lieu parallèlement aux directions x et y.

Dans l'invention on peut utiliser tout type de détecteur bidimensionnel, par exemple un capteur sensible aux rayons X et apte à fournir directement un signal électronique représentatif de l'image acquise par le détecteur sous forme de pixels.

Au lieu de cela on peut utiliser un écran-scintillateur prévu pour recevoir les rayons X ayant traversé le patient et pour convertir ces rayons X en lumière visible. Cette dernière est alors envoyée, par l'intermédiaire d'un miroir, à un capteur CCD muni d'un objectif et comprenant un réseau de pixels photosensibles.

Sur la figure 1, on voit aussi un dispositif 3 du genre contrôleur de CCD ou analogue qui lit, pixel par pixel, la représentation d'image fournie par le détecteur et qui numérise cette représentation. La représentation ainsi numérisée est stockée dans une mémoire 3a.

Un ordinateur 3b est prévu pour traiter les images ainsi mémorisées.

Un dispositif d'affichage 3c, comprenant par exemple un tube à rayons cathodiques, est prévu pour afficher les images avant ou après ce traitement.

Un tel système est donc utilisable pour mettre en oeuvre un procédé conforme à l'invention, selon lequel, en vue d'obtenir une bonne reproductibilité de la mesure de densité osseuse, on utilise un premier cliché, à faible dose, de type radioscopie, pour aider à positionner le patient dans le système d'ostéodensitométrie.

Revenons à l'utilisation de ce cliché de radioscopie pour le positionnement du patient.

Etant donné que, pour un système du genre "cone beam", on dispose d'un capteur bidimensionnel qui permet, en une seule acquisition, d'avoir une vision globale de la zone analysée, on propose, conformément à l'invention, de réaliser un cliché à faible dose (cliché de radioscopie) avant les acquisitions à haute et basse énergies pour aider au positionnement du patient.

Si ce patient est soumis à son premier examen, on utilise ce cliché de radioscopie pour rétroagir sur la mécanique du système (c'est-à-dire pour commander la mécanique du dispositif d'acquisition d'images de façon qu'il se positionne correctement par rapport au patient ou inversement) afin de positionner la zone anatomique par rapport à un référentiel préétabli.

Si l'examen est un examen de suivi du patient, on utilise le cliché de radioscopie pour mettre la zone anatomique dans une position identique à celle qu'elle occupait lors du précédent examen.

Ce type de démarche permet d'augmenter sensiblement la reproductibilité des mesures effectuées au moyen de systèmes de type "cone beam".

En raison de la conicité du faisceau de rayons X, la mesure dépend de la position de la zone anatomique dans ce faisceau.

En utilisant le cliché de radioscopie pour positionner de façon identique la zone anatomique du patient par rapport à un référentiel donné ou pour assurer la cohérence entre deux examens, on obtient une bonne reproductibilité de l'examen.

Un exemple de mise en oeuvre pour un patient soumis à son premier examen consiste à :
1. réaliser un cliché à faible dose ;
2. extraire de cette acquisition les contours des zones osseuses, l'extraction des contours étant réalisée par un logiciel par repérage des points de gradient maximum (ou de Laplacien nul) ;
3. repérer des points caractéristiques (ces points caractéristiques étant par exemple des points de forte courbure, des points d'inflexion ou des points d'intersection) dans la carte de contour, par exemple identification de vertèbres ou repérage de points caractéristiques sur le col du fémur, le repérage des points caractéristiques étant réalisé par un logiciel classique de traitement d'image ;
4. construire la fonction de type translation qui permet de placer au mieux ces points par rapport à une position standard définie au préalable, un logiciel déterminant (par exemple par une méthode de moindres carrés) les paramètres d'une translation permettant de ramener les points caractéristiques vers une position standard ;
5. rétroagir sur la mécanique de positionnement pour se ramener à la position standard ;
6. réaliser les acquisitions.

Dans le cas d'un patient soumis à un examen de suivi thérapeutique, l'étape de construction de fonction 4 est remplacée par les deux étapes suivantes :
4.1 récupérer les positions des points caractéristiques dans les acquisitions d'un examen précédent du patient ;
4.2 construire la fonction de type translation qui permet de mettre au mieux en correspondance les points caractéristiques du cliché de scopie par rapport à leur position dans un examen antérieur.

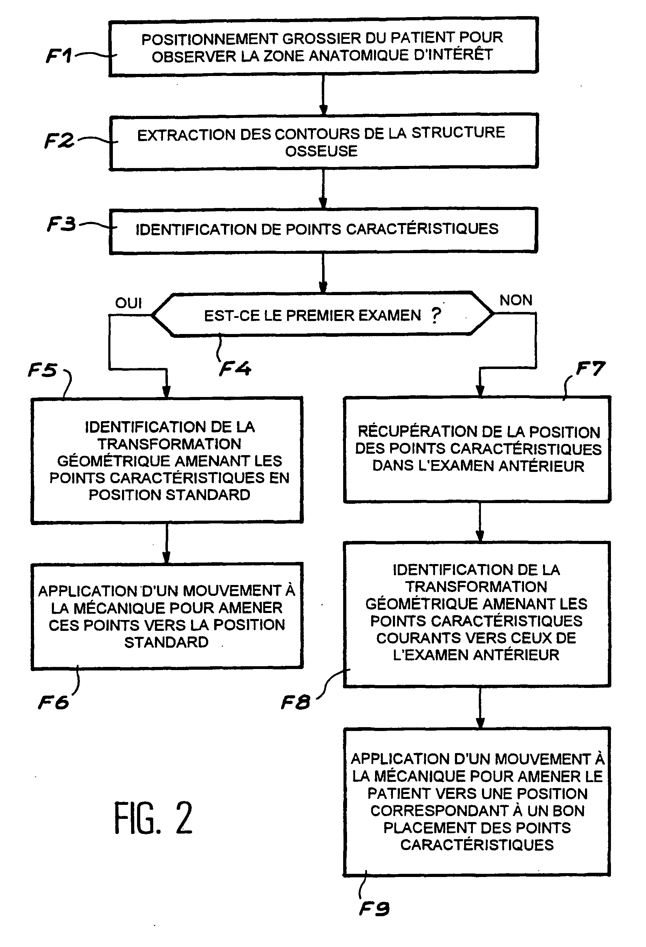

Tout ceci est précisé par l'organigramme de la figure 2 :
- Étape F1 : on positionne grossièrement le patient pour observer la zone anatomique d'intérêt (voir 1.)
- Étape F2 : on extrait les contours de la structure osseuse (voir 2.)
- Étape F3 : on identifie les points caractéristiques (voir 3.)
- Étape F4 : on se demande s'il s'agit du premier examen
- Si oui on va à l'étape F5 dans laquelle on identifie la transformation géométrique amenant les points caractéristiques en position standard (voir 4.) puis on va à l'étape F6 dans laquelle on applique un mouvement à la mécanique pour amener ces points vers la position standard (voir 5.)
- Si non on va à l'étape F7 dans laquelle on récupère la position des points caractéristiques dans l'examen antérieur (voir 4.1) puis on va à l'étape F8 dans laquelle on identifie la transformation géométrique amenant les points caractéristiques courants vers ceux de l'examen antérieur (voir 4.2) puis on va à l'étape F9 dans laquelle on applique un mouvement à la mécanique pour amener le patient vers une position correspondant à un bon placement des points caractéristiques (voir 5.).

Revenons au dispositif de la figure 1. On précise que la zone 1a de rayons X est munie de moyens de commande le permettant de faire varier la dose de rayons X transmise au patient 1c

Pour faire le cliché de radioscopie on règle ces moyens 1e pour que la source envoie une faible dose au patient, par exemple une dose égale à 1 µSv.

De plus, la source 1a est capable d'émettre des rayons X de basse énergie et des rayons X de haute énergie.

Pour faire le cliché de radioscopie, on utilise un faisceau de rayons X qui conduit à un bon contraste d'image et à une dose minimale pour le corps examiné, avec une énergie par exemple égale à 80 keV.

Le cliché de radioscopie est utilisable non seulement pour positionner le patient avant son examen mais encore pour optimiser la dose de rayons X qui sera transmise au patient lors de son examen.

Pour cette optimisation, on adapte le point de fonctionnement de la source à la morphologie (en particulier à l'épaisseur) du patient. On utilise dans ce cas le cliché de scopie pour déterminer l'ordre de grandeur de l'épaisseur du corps exposé.

En variante, pour optimiser la dose de rayons X qui sera transmise au patient lors de son examen, on ajuste la zone d'irradiation par les rayons X à la morphologie du patient. Dans ce cas, on utilise le cliché de radioscopie pour déterminer la zone osseuse et la zone de tissus autour de cette zone osseuse.

L'invention n'est pas limitée à l'amélioration d'un examen d'ostéodensitométrie. Elle s'applique à tout autre examen radiologique utilisant un détecteur bidimensionnel, par exemple un examen de suivi de consolidation de fracture osseuse..

De plus, l'invention n'est pas limitée à l'amélioration de l'examen radiologique d'un patient. Elle peut être mise en oeuvre avec tout corps vivant ou inerte, par exemple un tableau, avant de faire subir une radiographie à ce tableau.

## Revendications

1. Procédé d'amélioration d'un examen radiologique d'une zone d'un corps (1c), cet examen radiologique étant effectué au moyen d'un dispositif radiologique (1) comprenant un détecteur bidimensionnel (2) de rayons X, ce procédé étant **caractérisé en ce que**, avant l'examen radiologique, on fait un cliché bidimensionnel, de radioscopie, avec une seule énergie pour les rayons X utilisés, de la zone du corps à examiner et l'on utilise ce cliché pour déterminer des premiers points caractéristiques, qui définissent un référentiel de mesure, ainsi que des paramètres géométriques d'un déplacement apte à faire correspondre sensiblement ce référentiel de mesure avec un référentiel préétabli à partir de points caractéristiques équivalents correspondant aux premiers points caractéristiques.

2. Procédé selon la revendication 1, dans lequel l'examen radiologique est un examen d'ostéodensitométrie.

3. Procédé selon l'une quelconque des revendications 1 et 2, dans lequel l'examen radiologique est un premier examen radiologique et le référentiel préétabli est un référentiel théorique, adapté au corps examiné.

4. Procédé selon l'une quelconque des revendications 1 et 2, dans lequel l'examen radiologique suit un examen radiologique; précédent, effectué au moyen du même dispositif radiologique (1), et le référentiel préétabli est un référentiel qui est défini sur un cliché précédent à partir des points caractéristiques équivalents.

5. Procédé selon l'une quelconque des revendications 1 à 4, dans lequel on utilise en plus le cliché pour régler la dose de rayons X à transmettre au corps (1c) lors de l'examen radiologique.

6. Procédé selon l'une quelconque des revendications 1 à 5, dans lequel on règle la dose de rayons X à transmettre au corps (1c) lors de l'examen radiologique, en adaptant le point de fonctionnement du dispositif radiologique (1) à la morphologie du corps à examiner.

7. Procédé selon l'une quelconque des revendications 1 à 5, dans lequel on règle la dose de rayons X à transmettre au corps (1c) lors de l'examen radiologique, en ajustant, à la morphologie du corps à examiner, la zone à irradier par les rayons X lors de l'examen.

8. Dispositif radiologique pour la mise en oeuvre du procédé selon la revendication 1, ce dispositif (1) comprenant :
- une source (1a) de rayons X, apte à fournir un faisceau conique de rayons X à au moins une énergie,
- des moyens (1e) de variation de la dose de rayons X susceptible d'être reçue par le corps à examiner,
- un détecteur bidimensionnel (2) de rayons X, qui est disposé parallèlement à un plan défini par deux directions orthogonales (x, y) et qui est perpendiculaire à l'axe du faisceau de rayons X,
- des moyens (2a) de support du corps à examiner, ces moyens de support étant transparents aux rayons X émis par la source, disposés entre la source et le détecteur et susceptibles de subir un déplacement relatif par rapport à l'ensemble formé par la source et le détecteur, suivant les deux directions orthogonales (x, y),
- des moyens de détermination de premiers points caractéristiques sur un cliché bidimensionnel de radioscopie, ces premiers points définissant un référentiel de mesure, et
- des moyens de calcul, prévus pour déterminer les paramètres géométriques d'un déplacement apte à faire correspondre sensiblement ce référentiel de mesure avec un référentiel préétabli à partir de points caractéristiques équivalents correspondant aux premiers points caractéristiques.

## Claims

1. A process for improving a radiological examination of an area of a body (1c), this examination being conducted using a radiological device (1) comprising a two-dimensional X-ray detector (2), this process being **characterised in that**, prior to the radiological examination, a two-dimensional radioscopic image is taken, with a single energy for the X-rays used, of the area of the body to be examined and this image is used to determine first characteristic points which define a measurement reference system, and geometric parameters of a movement able to make this measurement reference system correspond approximately with a reference system pre-set from equivalent characteristic points corresponding to the first characteristic points.

2. A process according to claim 1, wherein the radiological examination is an osteodensitometry examination.

3. A process according to any one of claims 1 and 2, wherein the radiological examination is a first radiological examination and the pre-set reference system is a theoretical reference system, adapted to the body being examined.

4. A process according to any one of claims 1 and 2, wherein the radiological examination follows a previous radiological examination, conducted using the same radiological device (1), and the pre-set reference system is a reference system, which is defined on a previous image from the equivalent characteristic points.

5. A process according to any one of claims 1 to 4, wherein the image is additionally used to control the X-ray dose to be transmitted to the body (1c) during the radiological examination.

6. A process according to any one of claims 1 to 5, wherein the X-ray dose to be transmitted to the body (1c) is controlled during the radiological examination, by adapting the operating point of the radiological device (1) to the morphology of the body being examined.

7. A process according to any one of claims 1 to 5, wherein the X-ray dose to be transmitted to the body (1c) is controlled during the radiological examination, by setting the area to be irradiated by X-rays during the examination to the morphology of the body to be examined.

8. A radiological device for implementing the process according to claim 1, this device (1) including:
- an X-ray source (1a), able to supply an X-ray cone beam with at least one energy,
- means (1e) of varying the X-ray dose able to be received by the body to be examined,
- a two-dimensional X-ray detector (2), which is arranged parallel to a plane defined by two orthogonal directions (x, y) and which is perpendicular to the axis of the X-ray beam,
- means (2a) of supporting the body to be examined, these support means being transparent to the X-rays emitted by the source, arranged between the source and the detector and able to withstand a relative movement in relation to the unit formed by the source and the detector, along the two orthogonal directions (x, y),
- means of determining first characteristic points on a two-dimensional radioscopic image, these first points defining a measurement reference system, and
- calculation means, provided to determine the geometric parameters of a movement able to make this measurement system correspond approximately with a reference system pre-set from equivalent characteristic points corresponding to the first characteristic points.

## Patentansprüche

1. Verfahren zur Verbesserung einer radiologischen (bzw. Röntgenstrahl-) Untersuchung einer Zone eines Körpers (1c), unter Verwendung einer radiologischen (bzw. Röntgenstrahl-) Vorrichtung (1), welche einen zweidimensionalen Röntgenstrahl-Detektor umfasst, das Verfahren **dadurch gekennzeichnet, dass** man vor der radiologischen Untersuchung ein zweidimensionales Radioskopie-Cliché der zu untersuchenden Körperzone herstellt bei einer einzigen Energie für die hierbei verwendete Röntenstrahlung, und dass man diese Cliché verwendet zur Bestimmung erster chrakteristischer Punkte, welche ein Mess-Referenz definieren, sowie zur Bestimmung geometrischer Parameter einer Verschiebung, um diese Mess-Referenz im Wesentliche mit einer vorgegebenen Referenz in Entsprechung zu setzen, welche auf der Grundlage von den ersten charakterisierten Punkten entsprechenden äquivalenten charakteristischen Punkten aufgestellt wurde.

2. Verfahren nach Anspruch 1, bei welcher die radiologische (bzw. Röntenstrahl-) Untersuchung eine osteodensiometrische Untersuchung ist.

3. Verfahren nach einem der Ansprüche 1 und 2, bei welcher die radiologische Untersuchung eine erste radiologische Untersuchung und die vorgegebene Referenz eine dem untersuchten Körper angepasste Referenz ist.

4. Verfahren nach einem der Ansprüche 1 und 2, bei welcher die radiologische Untersuchung auf eine mit derselben radiologischen Vorrichtung vorgenommene vorhergehende radiologische Untersuchung folgt und die vorgegebene Referenz eine an einem vorhergehenden Cliché auf der Grundlage äquivalenter charakteristische Punkte definierte Referenz ist.

5. Verfahren nach einem der Ansprüche 1 und 4, bei welchem des Weiteren das Cliché zur Einstellregelung der Röntgenstrahldosis verwendet wird, mit welcher der Körper (1 c) bei der Untersuchung beaufschlagt wird.

6. Verfahren nach einem der Ansprüche 1 und 5, bei welchem man zur Einstellregelung der Röntgenstrahldosis für die Beaufschlagung des Körpers (1 c) bei der radiologischen Untersuchung den Arbeitspunkt der radiologischen (bzw. Röntgenstrahl-) Vorrichtung (1) der Morphologie des zu untersuchenden Körpers anpasst.

7. Verfahren nach einem der Ansprüche 1 und 5, bei welchem man zur Einstellregelung der Röntgenstrahldosis für die Beaufschlagung des Körpers (1c) bei der radiologischen Untersuchung, die bei der Untersuchung mit der Röntgenstrahlung zu bestrahlende Zone der Morphologie des zu untersuchenden Körpers anpasst.

8. Radiologische (bzw. Röntgenstrahl-) Vorrichtung zur Ausübung des Verfahrens nach Anspruch 1, wobei die Vorrichtung (1) umfasst:
- eine Röntgenstrahlquelle (1a) zur Erzeugung eines konischen Röntgenstrahlbündels mit wenigstens einer Energie,
- Mittel (1e) zur Änderung der Röntgenstrahldosis, mit welcher der zu untersuchende Körper beaufschlagt werden kann,
- einen zweidimensionalen Röntgenstrahldetektor (2), der parallel zu einer Ebene ist, die durch zwei zueinander rechtwinkelig Richtungen (x, y) definiert und senkrecht zur Achse des Röntgenstrahlbündels ist,
- Halterungsmittel (2a) für den zu untersuchenden Körper, die für die von der Strahlungsquelle emittierte Röntgenstrahlung transparent sind, zwischen der Strahlungsquelle und dem Detektor angeordnet sind, und eine eine Verschiebung entlang den beiden zueinander rechtwinkeligen Richtungen (x, y) relativ bezüglich dem aus Strahlungsquelle und Detektor bestehenden Ganzen erfahren können,
- Mittel zur Bestimmung erster charakteristischer Punkte auf einem zweidimensionalen Radioskopie-Cliché, wobei diese charakteristischen Punkte eine Mess-Referenz definieren, sowie
- Rechenmittel zur Bestimmung der geometrischen Parameter einer Verschiebung, um diese Mess-Referenz im Wesentlichen mit einer vorgegebenen Referenz in Entsprechung zu setzen, welche auf der Grundlage von den ersten charakteristischen Punkten entsprechenden äquivalenten charakteristischen Punkten aufgestellt wurde.
